# EUROPEAN PATENT APPLICATION

(11) **EP 4 530 914 A1**
(43) Date of publication of application: **02.04.2025**
(21) Application number: 23200955.5
(22) Date of filing: 29.09.2023
(51) Int. Cl.: G06F 30/27, G06F 30/28, G06N 3/044, G06N 3/045, G06F 111/10, G06F 113/08

(54) **TRANSIENT PREDICTIONS IN REACTING FLUID FLOW SIMULATIONS**

(71) Applicant: Siemens Aktiengesellschaft, 80333 München (DE)
(72) Inventor: MUKUNDAN, Meghesh, 560067 Bangalore (IN)
(74) Representative: Isarpatent

(57) **Abstract**

The present invention relates to transient predictions in reacting flow simulations. In one embodiment, the method (500) comprises generating an initial simulation of the reactive flows, by using a simulation technique, for a first time duration. The method (500) comprises predicting, by a machine learning model, a behavior of the reactive flows during a second time duration based on the initial simulation. The second time duration is consecutive to the first time duration. The method (500) comprises generating a subsequent simulation for the reactive flows for a subsequent time duration. The method (500) comprises providing data corresponding to the predicted behavior and the subsequent simulation as an input to the machine learning model to predict the behavior of the reactive flows for the subsequent time periods. In addition, the method (500) comprises repeating generating the subsequent simulation and predicting behavior of the reactive flows for one or more successive time durations, until the reaction of the reactive flows is completed.

## Description

The present invention generally relates to flow simulations, and more particularly relates to methods and systems for transient predictions in reacting fluid flow simulations.

Reacting fluid flow is a class of fluid flows where chemical reactions happen at the interphase between multiple fluids, within a single fluid, or at solid-liquid interphase. The reacting fluid flow involves multiple chemical reactions with heat release and heat absorption that results in production and consumption of multiple species/products. The physics involved in the reacting fluid flows is highly complex and transient, and the reacting fluid flows are dominated by different mechanisms (fluid dynamics, kinetics, interfaces, and structures) which exhibit instabilities.

One of the primary challenges while designing industrial applications with reacting flows is the design methodology. For example, most industrial equipments, such as mixing tanks, combustors, etc. have complex geometries and multiple inlets, fast chemistry, and high pressures. Designing such complex systems require a strong understanding of the physics and underlying mechanisms.

Lab scale testing and numerical simulation modeling are two approaches which are routinely used to gather insights about the physics and underlying mechanisms to provide design appropriate solutions. Lab scale testing facilitates testing a wide range of parameters and conditions, such as temperature, pressure, and pH, in a controlled environment. However, lab scale testing may be expensive and may offer limited macro-scale information about the behavior of the complex systems. Often times, it may be economically infeasible to conduct multiple experiments, as required in the product development and optimization phase.

Numerical simulation models, such as computational fluid dynamics (CFD), offer a cheaper alternative compared to lab scale testing. CFD is a very powerful and widely used numerical simulation method to study industrial flows. With advancement in chemical kinetics, turbulence and reacting flow modeling, CFD has found widespread usage in the simulation of reacting flows. However, the CFD simulations, especially for unsteady reacting flows, are highly compute-intensive and time consuming. For example, depending on size and nature of an industrial application, the simulation may take anywhere from a few hours to a few months of runtime in a high-performance computing (HPC) environment. As a result, involvement of CFD simulations in product development and optimization of industrial reacting flow problems is limited.

In light of the above, there exists a need to provide techniques for performing transient predictions of the reacting flows in reduced time and cost-effective manner.

Therefore, it is an object of the present invention to provide methods and computing systems for reducing the time required in simulating reactions of the reactive flows.

The object of the present invention is achieved by methods described herein. In a preferred embodiment of the present invention, the present invention describes a method for optimizing simulation of reactive flows. The method comprises generating an initial simulation of the reactive flows, by using a simulation technique, for a first time duration. In an example, the simulation technique may be a numerical simulation technique, such as a computation fluid dynamics (CFD) simulation technique.

Based on the initial simulation, the method includes predicting, by a machine learning model, a behavior of the reactive flows during a second time duration. In a preferred embodiment, the second time duration is consecutive to the first time duration. In an example, the machine learning model may be a recurrent neural network (RNN) model. Further, the method may include generating a subsequent simulation for the reactive flows, by using the simulation technique, for a subsequent time duration. The subsequent time duration is consecutive to the second time duration.

Further, the method includes providing data corresponding to the predicted behavior and the subsequent simulation as an input to the machine learning model to predict the behavior of the reactive flows for the subsequent time periods. determining whether or not a simulation of the reactive flows is completed. upon determination that simulation of the reactive flows is incomplete, repeating generating the subsequent simulation and predicting behavior of the reactive flows for one or more successive time durations, until the complete simulation of the reactive flows is achieved.

In a preferred embodiment, predicting the behavior of the reactive flows includes capturing a plurality of images pertaining to at least one region of interest during the first time duration. In an example, the plurality of images corresponds to a temperature contour of flow properties of the reactive flows. The plurality of images depicts different time frames of each pre-defined time duration.

In a preferred embodiment, generating the subsequent simulation for the subsequent time duration comprises determining that the predictions, predicted by the machine learning model are deviating from an actual behavior of the reactive flows after completion of the second time duration.

The present invention provides techniques for accelerating simulations of reactive fluid flows by employing simulation techniques in succession with machine learning model. Thus, simulations associated with unsteady fluid flows may be completed in lesser time period.

The object of the present invention is also achieved by a computing system for optimizing simulation of reactive flows. The computing system comprises one or more processing unit(s) and a memory coupled to the processing unit(s). The memory comprises modules stored in the form of machine-readable instructions executable by the processing unit. The modules are configured to perform the method as described above.

The object of the present invention is also achieved by a computer-program product having machine-readable instructions stored therein, that when executed by one or more processing unit(s), cause the one or more processing unit(s) to perform method steps as described above.

The object of the present invention is also achieved by a computer-readable medium which comprises a computer program code which when executed by a processing unit, causes the processing unit to carry out the method steps as described above.

The object of the present invention is also achieved by a cloud computing system for optimizing simulation of reactive flows. The cloud computing system comprises one or more processing unit(s) and at least one memory unit coupled to the one or more processing unit(s). The at least one memory unit comprises a simulation optimizing module stored in the form of machine-readable instructions executable by the processing units. The simulation optimization module is configured to perform the method as described above.

Advantageously, the simulation technique and the machine learning model when working in tandem results in significantly reducing the simulation runtime.

The above-mentioned and other features of the invention will now be addressed with reference to the accompanying drawings of the present invention. The illustrated embodiments are intended to illustrate, but not limit the invention.

The present invention is further described hereinafter with reference to illustrated embodiments shown in the accompanying drawings, in which:
FIG. 1 is a block diagram of a network environment for transient predictions in reacting fluid flow simulations, according to an embodiment of the present invention;
FIG. 2 is a Graphical User Interface showing an initial simulation of the reactive flow using a numerical simulation technique in a first time interval, according to an embodiment of the present invention;
FIG. 3 is a Graphical User Interface showing a prediction of the reactive fluid flow in a second time duration, according to an embodiment of the present invention;
FIG. 4 is a Graphical User Interface showing a plot depicting comparison in runtime involved in simulation techniques and machine learning model, according to an embodiment of the present invention;
FIG. 5 is a process flowchart illustrating an exemplary method of performing transient predictions in reacting fluid flow simulations, according to an embodiment of the present invention; and
FIG. 6 is a schematic representation of the cloud computing system, according to an embodiment.

Various embodiments are described with reference to the drawings, wherein like reference numerals are used to refer to the drawings, wherein like reference numerals are used to refer to like elements throughout. In the following description, for the purpose of explanation, numerous specific details are set forth in order to provide thorough understanding of one or more embodiments. It may be evident that such embodiments may be practiced without these specific details.

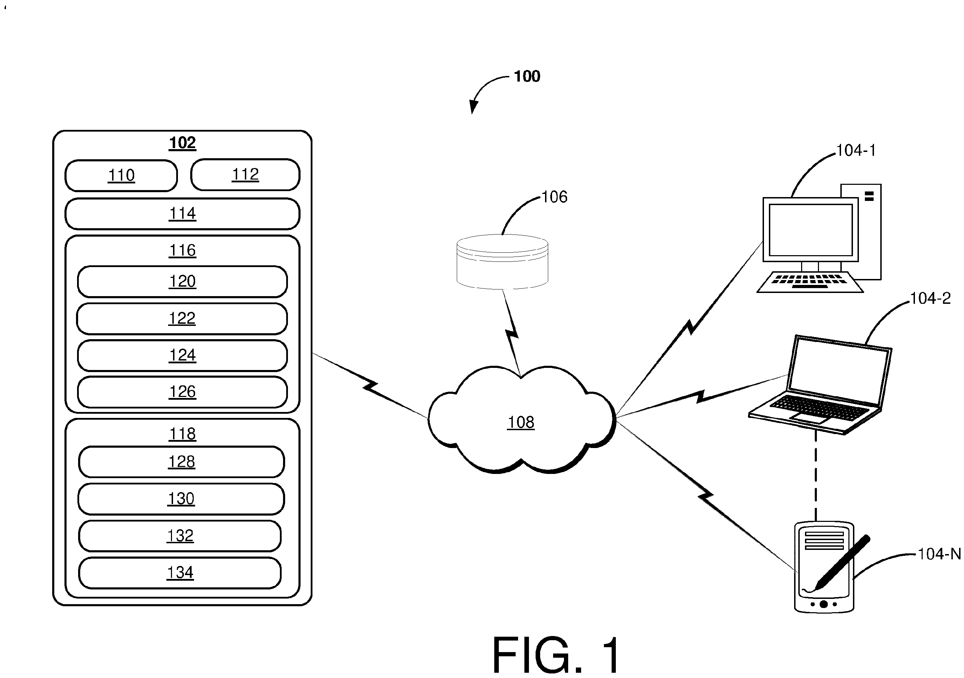

FIG. 1 is a block diagram of a network environment 100 for transient predictions in reacting fluid flow simulations, according to an embodiment of the present invention. Particularly, FIG. 1 depicts a computing system 102 which is capable of providing transient predictions in reacting fluid flow simulations in industrial applications. Examples of the computing system 102 may include, but are not limited to, a laptop, a notebook computer, a desktop computer. The computing system 102 may be a standalone server or may be a remote server on a cloud computing platform. In a preferred embodiment, the computing system 102 may be a cloud-based computing system.

The computing system 102 is connected to a plurality of user devices 104-1, 104-2,..., 104-N, collectively referred to as the user devices 104 and individually referred to as the user device 104. Examples of the user devices 104 may include, but are not limited to, a laptop, a notebook computer, a desktop computer, and a tablet. The network environment 100 further includes a database 106. The database 106 may communicate with the computing system 102 for storing the training data pertaining to various predictions of the reactive fluid flows.

In a preferred embodiment of the present invention, the computing system 102 is communicatively connected to the user devices 104 via a network 108. The network 108 may be a wireless network, a wired network, or a combination thereof. The network 108 can also be an individual network or a collection of many such individual networks, interconnected with each other and functioning as a single large network, e.g., the Internet or an intranet. The network 108 can be implemented as one of the different types of networks, such as intranet, local area network (LAN), wide area network (WAN), the internet, and such.

The computing system 102 may comprise processor(s) 110, interface(s) 112, and a memory 114. The processor(s) 110 may include microprocessors, microcomputers, microcontrollers, digital signal processors, central processing units, state machines, logic circuitries, and/or any other devices that manipulate signals and data based on computer-readable instructions. Further, functions of the various elements shown in the figures, including any functional blocks labelled as "processor(s)", may be provided through the use of dedicated hardware as well as hardware capable of executing computer-readable instructions.

The interface(s) 112 may allow the connection or coupling of the computing system 102 with one or more other devices, through a wired (e.g., Local Area Network, i.e., LAN) connection or through a wireless connection (e.g., Bluetooth^{®}, Wi-Fi). The interface(s) 112 may also enable intercommunication between different logical as well as hardware components of the computing system 102.

The memory(s) 114 may be a computer-readable medium, examples of which include volatile memory (e.g., RAM), and/or non-volatile memory (e.g., Erasable Programmable read-only memory, i.e., EPROM, flash memory, etc.). The memory(s) 114 may be an external memory, or internal memory, such as a flash drive, a compact disk drive, an external hard disk drive, or the like. The memory(s) 114 may further include data which either may be utilized or generated during the operation of the computing system 102.

The computing system 102 may further include engine(s) 116 and data 118. The engine(s) 116 includes a simulation engine 120, a prediction engine 122, a report generation engine 124, and other engine(s) 126. The other engine(s) 126 may further implement functionalities that supplement functions performed by the computing system 102 or any of the engine(s) 116. The data 118, on the other hand, includes data that is either stored or generated as a result of functions implemented by any of the engine(s) 116 or the computing system 102. It may be further noted that information stored and available in the data 118 may be utilized by the engine(s) 116 for performing various functions by the computing system 102. In an example, the data 118 may include simulation data 128, prediction data 130, anomaly data 132, and other data 134. It may be noted that such examples are only indicative. The present approaches may be applicable to other examples without deviating from the scope of the present subject matter.

The present invention facilitates in reducing the time involved in simulating reactive fluid flows, specifically in industrial applications. By employing both numerical simulation techniques and machine learning models, the simulation of reactive fluid flows is accelerated.

In operation, the simulation engine 120 may obtain data associated with the two reactive flows. Based on the data, the simulation engine 120 may generate an initial simulation of the reactive flows. In a preferred embodiment, the simulation engine 120 may employ numerical simulation techniques, such as a computational fluid dynamics (CFD) simulation, to generate the initial simulation of the behavior of the reactive flows. The behavior of the reactive flows may indicate flame and flow trajectories, flame dispersion, ignition timescale, flow field dynamics, and so on. In the CFD simulation, fluid flow and its associated physical properties, such as velocity, pressure, viscosity, density, and temperature, are calculated based on defined operating conditions. These quantities are calculated simultaneously to arrive at an accurate, physical solution. The CFD simulation uses a mathematical model and numerical method to predict the desired flow physics. For example, the CFD simulation techniques may be based on the Navier-Stokes (N-S) equations. The simulation engine 120 may store the simulations as simulation data 128.

In a preferred embodiment, the simulation engine 120 may perform the initial simulation for a first time duration. In an example, the first time duration may be pre-defined. For example, the first time duration may be for 50 milliseconds (ms), and so on. In case of 50 ms, the simulation engine 120 may simulate the reactive flows for t=1 ms to t=50 ms.

Further, during the initial simulation, a plurality of images pertaining to at least one region of interest may be captured. In an embodiment, the computing system 102 may employ high speed photography camera to capture the plurality of images, during the initial simulation. Each of the plurality of images may correspond to the behavior of the reactive flows at every instance of the first time duration. Referring to the earlier example, if the first time period includes 50 ms, the high-speed photography camera may capture 500 frames such that each image is captured at every 0.1 ms. Further, the plurality of images may be captured at a region of interest. The region of interest may refer to a specific plane of interest along which the simulations are to be observed.

In a preferred embodiment, the prediction engine 122 may provide the initial simulations obtained from the CFD technique as an input to a machine learning model. Based on the initial simulation, the machine learning model may predict, a behavior of the reactive flows during a second time duration. In an example, the prediction engine 122 may employ a ConvLSTM model to predict the behavior of the reactive flows in the second time duration. ConvLSTM model is a hybrid of a Convolutional Neural Network (CNN) and Long Short-Term Memory (LSTM) network.

In an example, prior to predicting the behavior of the reactive flows, the prediction engine 122 may train the machine learning model based on the simulations generated using the simulation technique. The ConvLSTM network consists of multiple ConvLSTM layers followed by batch normalization layers sandwiched together in different amounts. Each layer has varying parameters within followed by a 2D convolutional layer. The input dataset was split into 80% training and 20% validation. Once predicted, the accuracy of the prediction may be validated by visually comparing the predictions with simulations performed for the same time duration. The predictions of the ConvLSTM model which matches the simulations is considered for next steps and the predictions where deviation is observed with respect to the simulations, is discarded. The trained machine learning model may be stored in a memory, such as the memory 114. The prediction engine 122 may store information regarding any deviations or anomaly as the anomaly data 132.

In an example, the prediction engine 122 may feed the plurality of images captured during the first time duration as an input to the machine learning model to predict the behavior of the reactive flows for the second time duration. The second time duration is consecutive to the first time duration. Therefore, if the first time duration is of 50 ms, the second time duration may start from 51 ms. In an embodiment, the first time duration and the second time duration may be equal. Simulating the behavior of the reactive flows followed by predicting the behavior of the reactive flows results in reducing the time required to perform simulations of large scale applications.

In a preferred embodiment, the ConvLSTM model may, based on the input provided by the prediction engine 122, predict the subsequent behavior of the reactive flows. The predicted behavior is then compared with the simulations performed for the second time duration, to check if there is any anomaly in the predictions. If any anomaly is observed, the predictions that do not have the anomaly are considered for performing the simulations.

Once the behavior is predicted for the second time duration, the simulation engine 120 may generate a subsequent simulation for the reactive flows by using the simulation technique. In an example, the subsequent simulation is performed for a time duration subsequent to the second time duration. In one implementation, if the correct predictions were observed from the machine learning model for 10 ms, the subsequent time duration would be consecutive to 50 ms (first time duration) + 10 ms (second time duration), i.e., 60 ms. In another implementation, the simulation and prediction may be alternated after every 10 ms.

In a preferred embodiment, the prediction engine 122 may provide data corresponding to the predicted behavior and the subsequent simulation as an input to the machine learning model to predict the behavior of the reactive flows for the subsequent time periods. Thus, an input dataset being provided to the machine learning model is increased to facilitate the machine learning model to provide accurate predictions.

In a preferred embodiment, the simulation engine 120 may determine if the reaction between the reactive flows is completed or not. Upon determination that the reaction of the reactive flows is incomplete, the simulation engine 120 may generate the subsequent simulation for one or more successive time durations. Thereafter, the prediction engine 122 may predict behavior of the reactive flows using the simulations as the input, until the reaction of the reactive flows is completed.

Upon completion of the reaction, the report generation engine 124 may generate a report based on the simulation and prediction results. For example, the report may depict the time saved by alternating between the simulation and predictions. The report may also indicate the type of reacting flows for which the simulation is performed.

Accordingly, the present invention facilitates in saving cost and time while accelerating reactive flow simulation, especially for industrial applications. The ConvLSTM model coupled with CFD simulation ensures that the results of the simulations are accurate at the same time speeding up the simulations.

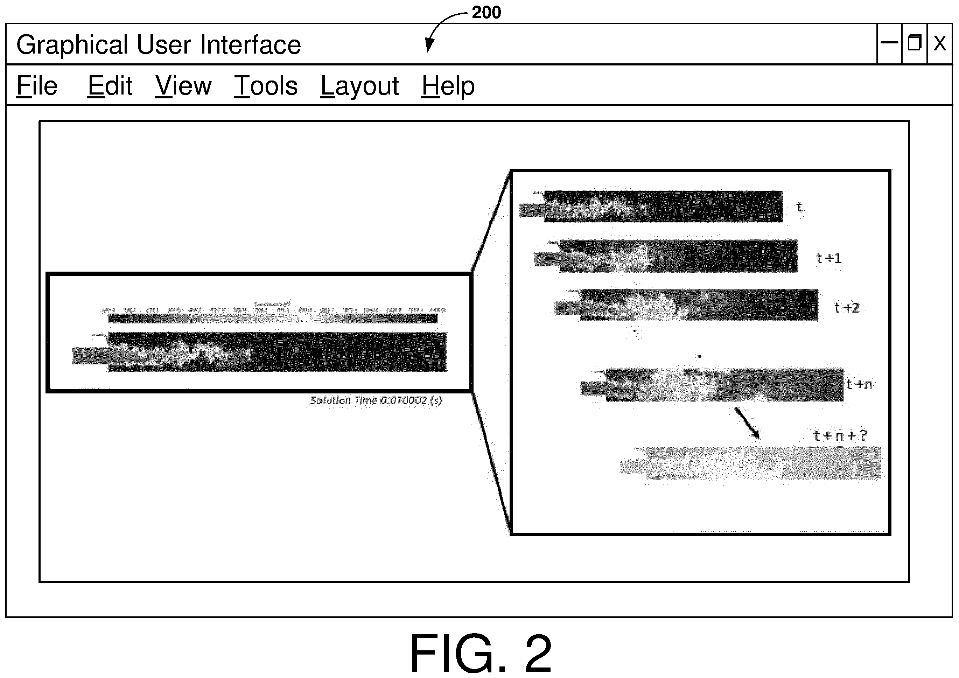

FIG. 2 is a Graphical User Interface (GUI) 200 showing an initial simulation of the reactive flow using a numerical simulation technique in a first time duration, according to an embodiment of the present invention. As described above, the reaction of the reactive flows is simulated using numerical simulation technique, such as the CFD technique for the first time duration. In an example, the first time duration is pre-defined, such as 50 ms. As depicted in FIG. 2, the simulations represent behavior of unsteady, reacting flows, subject to an operating condition. The GUI 200 depicts a plurality of images captured over the first time duration, during the initial simulation. The plurality of images may be captured for at least one region of interest.

For example, in a preferred embodiment, the plurality of images may be captured to gather temperature contour of flow properties (P, T, V) and species concentrations (O₂, CO₂, CO, H₂O) of the reactive flows. In an example, the plurality of images may be captured through a high speed photographic camera. Each of the plurality of images corresponds to behavior of the reactive flows at every instance of the first time duration. For example, in the 50 ms duration, 500 images may be captured such that each image is captured at an interval of 0.1 ms. This data is collected at multiple time intervals during the course of simulation (say, t0, t1, t2, ...., tn).

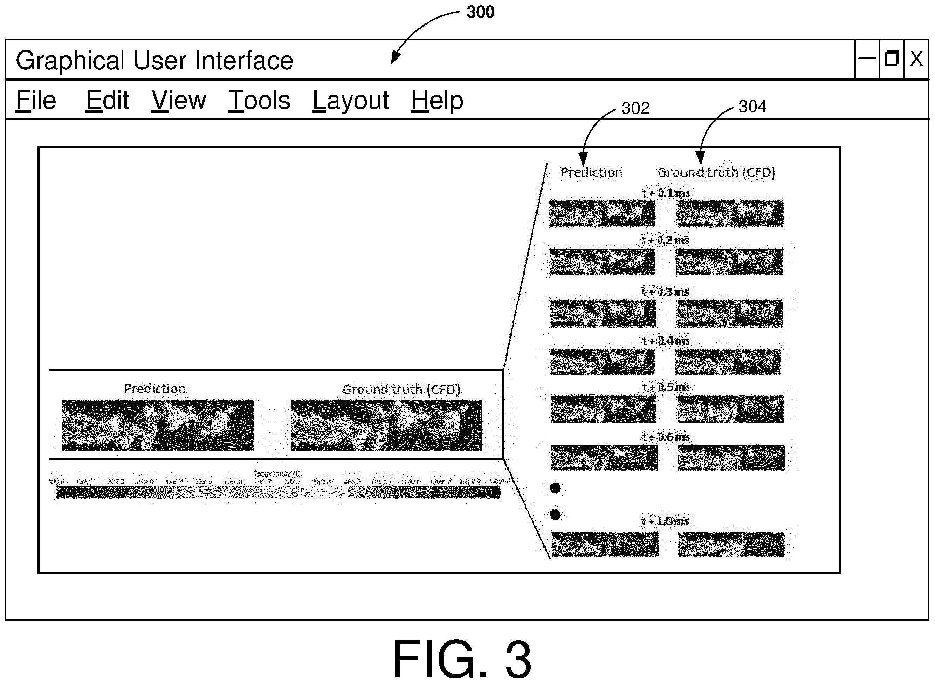

FIG. 3 is a Graphical User Interface 300 showing a prediction of the reactive flows in a second time duration, according to an embodiment of the present invention. In a preferred embodiment of the present invention, a machine learning model, such as a ConvLSTM model is employed to predict the behavior of the reactive flows in a second time duration. ConvLSTM model is a hybrid of a Convolutional Neural Network (CNN) and Long Short-Term Memory (LSTM) network. CNN is a type of neural network that is well suited for extracting and co-relating features from images. LSTM network is a type of Recurrent Neural Network (RNN) which is well suited for time series prediction. ConvLSTM models combine the powerful capabilities of the CNN & LSTM framework and are well suited for predicting reacting flows with spatially & temporally evolving flow behavior.

As depicted in FIG. 3, the plurality of images captured during the first time duration are provided as an input to the machine learning model to predict the behavior of the reactive flows for the second time duration. The second time duration is consecutive to the first time duration. For example, the 500 images corresponding to the simulation of 50 ms is provided as the input to the ConvLSTM model to predict the behavior of the reactive flows for the second time duration. The ConvLSTM model may, based on the input, predict the subsequent frames or images of the transient flow of the reactive flows. The predicted behavior 302 is then compared with the simulations 304 performed for the second time duration, to check if there is any anomaly in the predictions. The predictions are performed for the same properties that are provided as training data. For example, as temperature contour images were provided as an input to the ConvLSTM model, the ConvLSTM model may predict the temperature contour of the reacting flows in the subsequent frames. Likewise, other flow or reactive physics properties, such as Pressure, velocity, species concentrations, etc., may be provided as the input to get corresponding predictions. As can be seen from FIG. 3, after a few milliseconds, there is a deviation in the predictions and the simulations. Thus, the time period till which the predicted behavior 302 is similar to the simulations 304, is taken into consideration for the subsequent simulations.

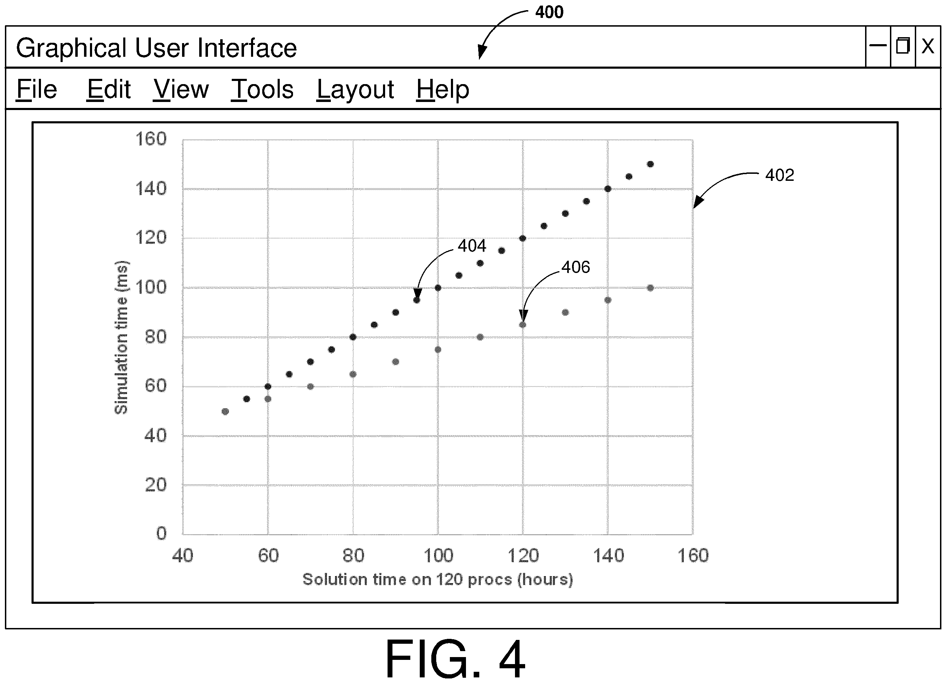

FIG. 4 is a Graphical User Interface 400 showing a plot 402 depicting comparison in runtime involved in simulation techniques and machine learning model, according to an embodiment of the present invention. The plot 402 includes a line 404 to indicate the runtime involved in prediction and a line 406 to indicate the runtime involved in simulation. As may be seen, the use of the ConvLSTM model in a coupled manner with the CFD simulation technique may reduce the simulation runtimes significantly.

For example, referring again to the above example, simulating for 50 milliseconds (ms) with a time step of 0.1 microsecond (µs) on 120 CPUs may take approximately 50 hours. This means, about 1 hour is required to progress through 1 ms of simulation. For a good, converged solution, the simulation needs to be run for at least 150 ms (-150 hours clock time). Assuming that after the first 50 ms of CFD simulation, the process is switched between ConvLSTM prediction and CFD simulation every 5 ms. Such an alternate arrangement results in at least a 33% speedup of the simulation process.

Advantageously, the present invention reduces costs involved in product development & optimization phases by accelerating the simulations of the reactive flows using machine learning model and numerical simulation techniques.

Those skilled in the art will recognize that, for simplicity and clarity, the full structure and operation of all data processing systems suitable for use with the present disclosure is not being depicted or described herein. Instead, only so much of a computing system 102 as is unique to the present disclosure or necessary for an understanding of the present disclosure is depicted and described. The remainder of the construction and operation of the computing system 102 may conform to any of the various current implementation and practices known in the art.

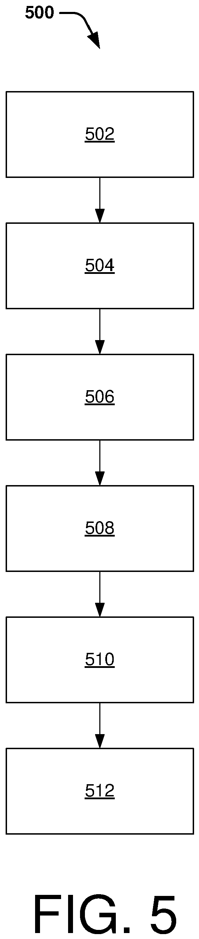

FIG. 5 is a process flowchart illustrating an exemplary method 500 of performing transient predictions in reacting fluid flow simulations, according to an embodiment of the present invention.

At step 502, the method 500 includes generating an initial simulation of the reactive flows for a first time duration. In an example, the initial simulation is generated using a simulation technique, such as a numerical simulation technique. In a preferred embodiment, the numerical simulation technique is a computational fluid dynamics (CFD) simulation technique. The CFD simulation technique may be implemented by a simulation engine 120 of the computing system 102 to simulate the mixing process of multiple fluids within any given mixing body. The geometry of the mixing body may be represented by a digital three-dimensional (3D) model. The digital 3D model may be in the form of an electronic file for Computer Aided Design (CAD), Computer Aided Manufacturing (CAM), Computer Aided Engineering (CAE), or other suitable application.

The digital 3D model of the mixing body, along with other simulation model parameters, may be provided to a computer-implemented simulation method to simulate the process of mixing reactive fluid flows. The simulation results may be further processed to generate a spatial distribution of fluid concentrations of the mixture.

In a preferred embodiment, the first time duration for performing the initial simulation is 50 milliseconds (ms). During the initial simulation, a plurality of images may be captured pertaining to at least one region of interest during the first time period. The plurality of images may be captured by a high speed photography camera of the computing system 102. Each of the plurality of images may correspond to the behavior of the reactive flows at every instance of the first time duration. For example, in the 50 ms duration, 500 images may be captured. Further, the plurality of images corresponds to a temperature contour of flow properties, such as pressure (P), temperature (T), volume (V), and species concentration (O₂, CO₂, H₂O) of the reactive flows.

At step 504, based on the initial simulation, a behavior of the reactive flows is predicted during a second time duration. In an example, the prediction engine 122 may employ a machine learning model to predict the behavior of the reactive flows for the second time duration. In an example, the second time duration is consecutive to the first time duration. In a preferred embodiment, the plurality of images captured during the initial simulation may be fed to the machine learning model. In an example, the machine learning model is a recurrent neural network (RNN) model. Specifically, the RNN model is a convolutional Long Short-Term Memory (ConvLSTM) model. The plurality of images is fed to the ConvLSTM model to extract the features pertaining to the plurality of images and predict a future frame. For example, based on the plurality of images, the ConvLSTM model may extract features, such as flow trajectory, flame dispersion, mixing. Based on the extracted features, the ConvLSTM model may make the predictions for the future frames.

For a sequence of images, the ConvLSTM combines the features and advantages of LSTM and CNN. The prediction engine 122 may store the output of the predictions as the prediction data 130. Although the present invention is described with reference to ConvLSTM model, it would be understood to a person skilled in the art that any other machine learning models may be employed by the prediction engine 122, either alone or in combination with the ConvLSTM model to predict the behavior of the reacting flows.

In an example, prior to predicting the behavior of the reactive flows, the machine learning model may be trained based on the simulations generated using the simulation technique. The ConvLSTM network consists of multiple ConvLSTM layers followed by batch normalization layers sandwiched together in different amounts. Each layer has varying parameters within followed by a 2D convolutional layer. The input dataset was split into 80% training and 20% validation. Once predicted, the accuracy of the prediction may be validated by visually comparing the predictions with simulations performed for the same time duration. The output of the ConvLSTM model which matches the simulations is considered for next steps and the output where deviation is observed with respect to the simulations, is discarded. The trained machine learning model may be stored in a memory, such as the memory 114. The prediction engine 122 may store information regarding any deviations or anomaly as the anomaly data 132.

At step 506, a subsequent simulation is generated for the reactive flows for a subsequent time duration. It will be evident to a person skilled in the art that the subsequent simulations are performed by the simulation technique and the subsequent time duration is consecutive to the second time period. In an implementation, the processor(s) 110 may switch between the simulations using the numerical techniques and the predictions using the machine learning model to reduce the time involved in simulating complex problems. The simulation engine 120 may store the output of the simulations as the simulation data 128.

At step 508, data corresponding to the predicted behavior and the subsequent simulation is provided as an input to the machine learning model to predict the behavior of the reactive flows for the subsequent time periods. Thus, the prediction engine 122 may dynamically train the machine learning model by feeding large datasets for the machine learning model to predict in an accurate manner. Thus, every subsequent prediction, the machine learning model would be provided with not just the data of a previous simulation but the data pertaining to a previous prediction as an input.

At step 510, it is determined whether or not a reaction of the reactive flows is completed. For example, based on the flow properties and species concentration, the processor(s) 110 may determine if the reaction of the reactive flows is complete or not.

At step 512, upon determination that simulation of the reactive flows is incomplete, the subsequent simulation and prediction of behavior of the reactive flows is repeated for one or more successive time durations, until the complete simulation of the reactive flows is achieved.

Advantageously, the use of ConvLSTM model in a coupled manner with the CFD simulation technique may significantly reduce the time involved in performing simulations of the behavior of the reactive flows, especially unsteady reactive flows in various industrial applications.

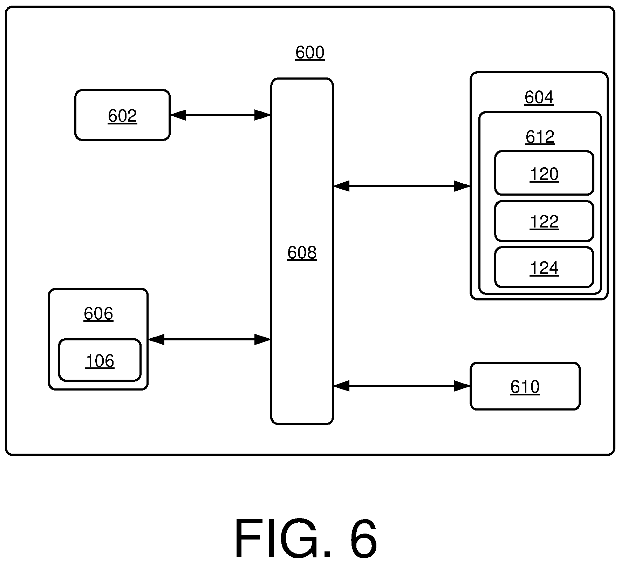

FIG. 6 is a schematic representation of the cloud computing system 600, according to an embodiment of the present invention. The cloud computing system 600 comprises processing units 602, a memory unit 604, a storage unit 606, a communication interface 608, and a cloud interface 610.

The processing units 602 may be one or more processors (e.g., servers). The processing units 602 is capable of executing machine-readable instructions stored on a computer-readable storage medium such as the memory unit 604 for performing one or more functionalities described in the foregoing description including but not limited to optimizing simulation of reactive flows. The memory unit 604 comprises a cloud computing platform 612 stored in the form of machine-readable instructions and executable by the processing units 602. Alternatively, the cloud computing platform 612 may take form of hardware such as a processor with embedded software. The cloud computing platform 612 comprises the simulation engine 120, the prediction engine 122, and the report generation engine 124 stored in the form of machine- readable instructions executable by the processing units 602.

The storage unit 606 can be volatile or non-volatile storage. In the preferred embodiment, the storage unit 606 comprises the database 106 for storing the training data pertaining to various predictions of the reactive fluid flows. The storage unit 606 may also store untrained data received from the predictions of the reactive flows. The communication interface 608 acts as an interconnect means between different components of the cloud computing system 600. The communication interface 608 may enable communication between the processing units 602, the memory unit 604 and the storage units 606. The processing units 602, the memory unit 604 and the storage unit 606 may be located in same location or at different locations.

The cloud interface 610 is configured to establish and maintain communication links with the industrial application. Also, the cloud interface 610 is configured to maintain a communication channel between the cloud computing system 600 and the user devices 104.

In various embodiments, the cloud computing system 600 provides a platform for transient predictions of different reacting fluid flow simulations. The cloud computing system 600 enables simulating and predicting behavior of the reactive fluid flows for being used in various industrial applications, such as mixing tanks, combustors, and so on. The cloud computing system 600 combines the numerical simulation techniques with predictions of machine learning models to reduce the overall time required in simulating the behavior of the reactive fluid flows.

The present invention can take a form of a computer program product comprising program modules accessible from computer-usable or computer-readable medium storing program code for use by or in connection with one or more computers, processing units, or instruction execution system. For the purpose of this description, a computer-usable or computer-readable medium can be any apparatus that can contain, store, communicate, propagate, or transport the program for use by or in connection with the instruction execution system, apparatus, or device. The medium can be electronic, magnetic, optical, electromagnetic, infrared, or semiconductor system (or apparatus or device) or a propagation mediums in and of themselves as signal carriers are not included in the definition of physical computer-readable medium include a semiconductor or solid state memory, magnetic tape, a removable computer diskette, random access memory (RAM), a read only memory (ROM), a rigid magnetic disk and optical disk such as compact disk read-only memory (CD-ROM), compact disk read/write, and DVD. Both processing units and program code for implementing each aspect of the technology can be centralized or distributed (or a combination thereof) as known to those skilled in the art.

While the present invention has been described in detail with reference to certain embodiments, it should be appreciated that the present invention is not limited to those embodiments. In view of the present disclosure, many modifications and variations would be present themselves, to those skilled in the art without departing from the scope of the various embodiments of the present invention, as described herein. The scope of the present invention is, therefore, indicated by the following claims rather than by the foregoing description. All changes, modifications, and variations coming within the meaning and range of equivalency of the claims are to be considered within their scope. All advantageous embodiments claimed in method claims may also be apply to system/apparatus claims.

### List of Reference Numerals:

1. a network environment 100
2. a computing system 102
3. user devices 104
4. a database 106
5. a network 108
6. processor(s) 110
7. interface(s) 112
8. a memory 114
9. engine(s) 116
10. data 118
11. a simulation engine 120
12. a prediction engine 122
13. a report generation engine 124
14. other engine(s) 126
15. simulation data 128
16. prediction data 130
17. anomaly data 132
18. other data 134
19. a Graphical User Interface 200
20. a Graphical User Interface 300
21. predicted behavior 302
22. simulations 304
23. Graphical User Interface 400
24. a plot 402
25. a line 404
26. a line 406
27. a method 500
28. method steps 502-512
29. cloud computing system 600
30. processing units 602
31. a memory unit 604
32. a storage unit 606
33. a communication interface 608
34. a cloud interface 610
35. a cloud computing platform 612

## Claims

1. A method (500) for optimizing simulation of reactive flows, the method (500), executed by a processing unit (110, 602), comprising:
generating an initial simulation of the reactive flows, by using a simulation technique, for a first time duration;
based on the initial simulation, predicting, by a machine learning model, a behavior of the reactive flows during a second time duration, wherein the second time duration is consecutive to the first time duration;
generating a subsequent simulation for the reactive flows, by using the simulation technique, for a subsequent time duration;
providing data corresponding to the predicted behavior and the subsequent simulation as an input to the machine learning model to predict the behavior of the reactive flows for the subsequent time periods;
determining whether or not a reaction between the reactive flows is completed; and
upon determination that the reaction of the reactive flows is incomplete, repeating generating the subsequent simulation and predicting behavior of the reactive flows for one or more successive time durations, until the reaction of the reactive flows is completed.

2. The method (500) according to claim 1, wherein predicting the behavior of the reactive flows further comprises:
during each generated simulation, capturing a plurality of images pertaining to at least one region of interest during the first time duration.

3. The method (500) according to claim 1 or claim 2, wherein the plurality of images corresponds to a temperature contour of flow properties of the reactive flows.

4. The method (500) according to claims 1 to 3, wherein each of the plurality of images corresponds to behavior of the reactive flows at every instance of the first time duration.

5. The method (500) according to one of claims 1 to 4, wherein generating the subsequent simulation for the subsequent time duration further comprises:
determining that the predictions, made by the machine learning model, are deviating from an actual behavior of the reactive flows after completion of the second time duration.

6. The method (500) according to one of claims 1 to 5, wherein the method further comprises:
based on the determination, discarding the predictions that are deviating from the actual behavior of the reactive flows.

7. The method (500) according to claim 1, wherein the simulation technique is a computational fluid dynamics (CFD) technique.

8. The method according (500) to claim 1, wherein the method further comprises generating a report based on the simulation and prediction results.

9. The method (500) according to claim 1, wherein the machine learning model is a recurrent neural network (RNN) model.

10. The method (500) according to claim 9, wherein the RNN model is a convolutional Long Short-Term Memory (ConvLSTM) model.

11. The method (500) according to claim 1, wherein the method comprises training the machine learning model based on the simulations generated using the simulation technique for the reactive flows, prior to predicting the behavior of the reactive flows.

12. A computing system (102) having a processing unit (110) to execute a method according to one of claims 1 to 11.

13. A computer-program product, having computer program code which, when executed by a processing unit, causes the processing unit to carry out the method according to one of claims 1 to 11.

14. A computer-readable medium comprising a computer program product comprising computer program code which, when executed by a processing unit, causes the processing unit to carry out the method according to one of claims 1 to 11.

15. A cloud computing system (600) comprising:
one or more processing units (602); and
at least one memory unit (604) communicatively coupled to the one or more processing units (602), wherein the at least one memory unit (604) comprises a simulation optimizing module stored in the form of machine-readable instructions executable by the one or more processing units, and wherein the simulation optimizing module is configured to perform or initiate a method according to one of claims 1 to 11.
